# EUROPEAN PATENT SPECIFICATION

(11) **EP 0 467 088 B1**
(45) Date of publication and mention of the grant of the patent: **20.04.1994**
(21) Application number: 91110114.5
(22) Date of filing: 20.06.1991
(51) Int. Cl.: A61B 17/12, A44B 11/25, A44C 11/02

(54) **Closure for ends of band or the like**
Verschluss für Bandenden oder dergleichen
Fermoir pour extrémités d'une bande ou analogues

(30) Priority: 21.06.1990 DE 9006941 U
(43) Date of publication of application: 22.01.1992
(73) Proprietor: HOLTSCH Metallwarenherstellung Maria Holtsch, D-65232 Taunusstein (DE)
(72) Inventor: Holtsch, Peter E., W-6204 Taunusstein (DE)

(56) References cited:
- EP-A- 0 305 130
- DE-U- 8 708 299
- GB-A- 2 169 950

## Description

The present invention relates to a closure for opposite end of a band or the like. More particularly, it relates to such a closure which has a closure housing with a projecting engaging element for a housing cap in which the end of a band is mounted, and a push button device for releasing the housing cap from the closure housing.

Closures of the above mentioned general type are known in the art. One of such closures is disclosed in the German document DE-GM 8,708,299.3 as applied to a turniquet for limbs. The above mentioned document describes a closure in which the arresting element has a central, springy ledge and two inclined outwardly oriented, springy or spring-elastic hooks with outwardly oriented projections. The ledge engages in a central guide of the cap provided on the band end during connecting and engaging of the housing cap with a closure housing. Two convex sliding surfaces facing the interior of the cap are arranged on the cap symmetrically relative to its central axis for guiding purposes. The distance between the sliding surfaces is smaller than the distance of the outer surfaces of the hooks. The sliding surfaces extend to the height of two lateral push buttons and each form there a step, behind which the projection engages in the locked condition of the cap. The push buttons have inclined surfaces on their inner ends. During opposite pressing of the push buttons the inclined surfaces extend flush with the convex sliding surfaces, so that in each case two cooperating sliding surfaces are produced for the springy hook. SB-A-2169 950 discloses a closure for a band and the like, comprising a closure housing adapted for passing a band therethrough; a housing cap adapted to be connected to an end of the band; and means for releasably connecting said closure housing with said housing cap, said connecting means including a pin projecting from the closure housing and having a front end with two oppositely inclined surfaces and two sliders slidingly accommodated in said housing cap so as to slide one over the other

The above described closure operates in a satisfactory manner. However, it has the disadvantage that the central projecting ledge and two spring-elastic lateral hooks can be broken as a result of careless manipulations, and the lock becomes unuseable. Furthermore it has the disadvantage that the sliders are provided with separate elastic springs.

### SUMMARY OF THE INVENTION

Accordingly, it is an object of the present invention to provide a closure of the above mentioned general type, which not only provides a fast and reliable connection of the closure housing with the housing cap, but also has a special construction of their connecting means characterized by high safety against breaking of its parts.

This object is solved by the characterizing part of claim 1.

In keeping with these objects and with others which will become apparent hereinafter, the present invention resides, briefly stated, in a closure which has a pin extending from the outside of the closure housing and having a front end provided with two oppositely inclined surfaces and also two transverse grooves, while the housing cap has two superposed and oppositely movable sliders each having a projection and a shaped formation formed so that in a locked condition of the closure housing and the housing cap the projections of the sliders engage in the grooves of the pin while the shaped formation of the sliders abut against the inclined surfaces of the pin.

When the closure is designed in accordance with the present invention, the single pin extending from the closure housing provides for a firm and reliable cooperation and extraordinarily strong connection with the housing cap. Moreover, when in accordance with another feature of the present invention the pin has a rectangular cross-section, its practically impossible to break the pin. Due to the special construction of the oppositely movable sliders arranged in the housing cap, a reliable arresting of the pin without additional springs is achieved. Also, by the abutment of the shaped formations on the opposite inclined surfaces, the arresting of the pin is maintained. By pressing on the push buttons, the pin after the disengagement of the projections from the grooves is pushed outwardly. Therefore a very efficient opening of the closure is achieved. The other preferred embodiments are according to the dependent claims 2-10.

The invention itself, however, both as to its construction and its method of operation, together with additional objects and advantages thereof, will be best understood from the following description of specific embodiments when read in connection with the accompanying drawings.

### BRIEF DESCRIPTION OF THE DRAWINGS

FIG. 1 is a side view of a closure in accordance with the present invention;
FIG. 2 is a plan view of the closure shown in FIG. 1, with a removed cover of a housing cap;

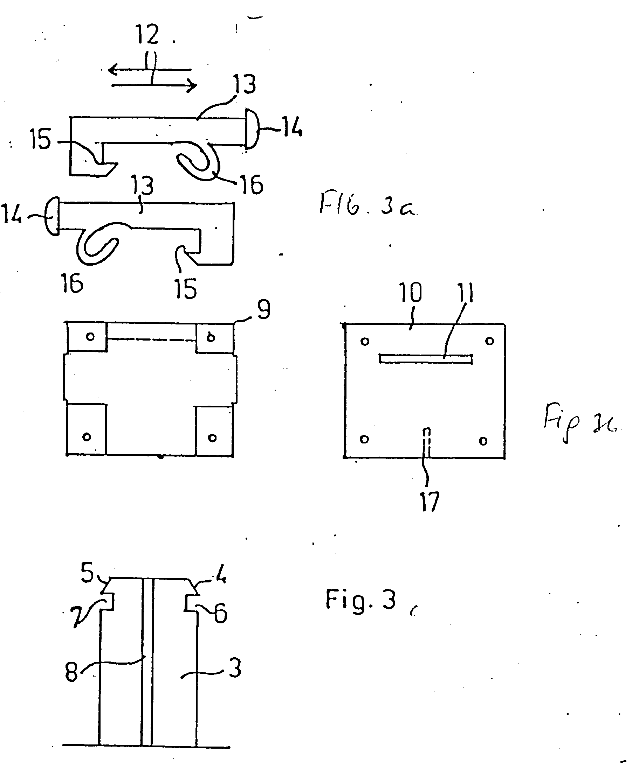

FIG. 3A is a view showing two sliders of connecting means for connecting a closure housing with a housing cap;
FIG. 3B is a view showing a shaped part and a cover of a housing cap;
FIG. 3C is a view showing a pin projecting from the closure housing;
FIG. 4A is a view substantially corresponding to the view of FIG. 3A, but showing two sliders in accordance with a further modification of the present invention; and
FIG. 4B is a view showing a pin projecting from the closure housing and cooperating with the sliders of FIG. 4A in accordance with the other embodiment of the invention.

### DESCRIPTION OF A PREFERRED EMBODIMENT

A closure for a band which is used as an example, for a turniquet for limbs in accordance with a present invention has a closure housing which is identified with reference numeral 1 and provided with a slot in its lower part. A band 2 is passed through the slot and forms a loop. A not shown mechanism is accommodated in the interior of the housing 1 and is actuatable by a button so that when the band is wound around a limb to be compressed the band 2 is fixed.

A pin 3 extends from the closure housing 1 and is fixedly connected with it. The pin 3 is inclined relative to the housing and has a rectangular cross-section. The pin 3 has a front end which is provided with two oppositely inclined surfaces 4 and 5 which together with an end surface of the pin form a roof-shaped profile. Two transverse grooves 6 and 7 are located between the front end of the pin 3 provided with the inclined surfaces 4 and 5, and the remaining portion of the pin 3. The pin 3 further has a longitudinal groove 8 which extends in a longitudinal direction.

The closure further has a housing cap composed substantially of two parts. The first part of the housing cap is a rectangularly shaped part 9 of synthetic plastic material, while the second part is a cover 10 which is screwed or riveted on the shaped part 9. The cover 10 has a slot 11 for the passage of the band 2. Two sliders 13 are arranged in the shaped part 9 of the housing cap. The sliders 13 are located over one another and movable relative to one another in direction of the double arrow 12. The sliders 13 are identical and engage with the pin 3.

Each sliders 13 has a first end provided with a push button 14 and a second end provided with a projection 15. The projection 15 is transversely offset relative to the main portion of the slider 13 and extend in direction toward the push button 14. In the arrested condition of the closure housing 1 with the housing cap or more particularly of the pin 3 with the sliders, the projections 15 of the sliders 13 engage in the respective grooves 6 and 7.

An ear-like bent hook 16 is arranged between the push button 14 and the projection 15 of each slider 13. In the arrested position, the hook 16 abuts against the respective, oppositely located upper inclined surface 4 or 5 of the pin 3. A guiding lug 17 is arranged on the inner side of the cover 10. The guiding lug 17 engages in the longitudinal groove 8 of the pin 3.

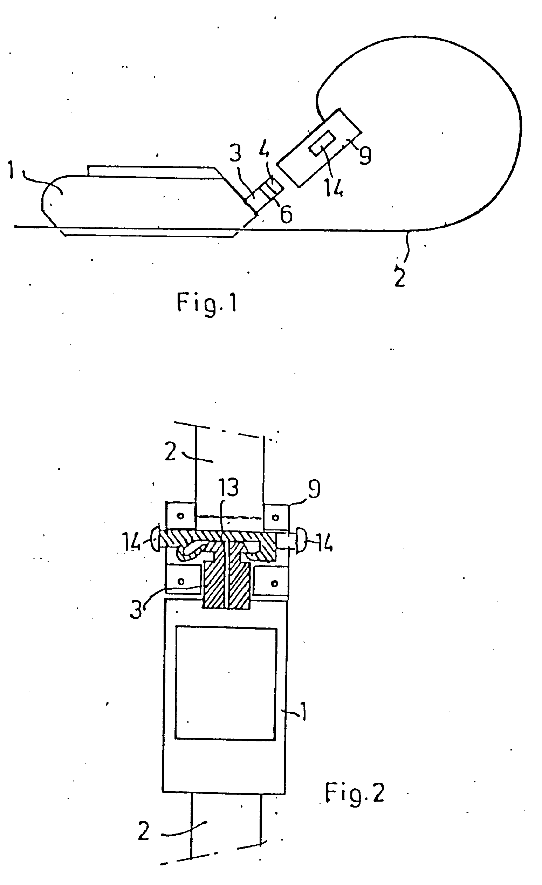

In the arrested condition shown in FIG. 1 the projections 15 of the sliders accommodated in the housing cap engage in the grooves 6 and 7 of the pin 3 of the closure housing 1. On the other side, the hooks 16 of the sliders 13 accommodated in the housing cap abut against the inclined surfaces 4 and 5 of the pin 3 of the closure housing 1.

For releasing the housing cap from the pin 3 and therefore from the closure housing, an opposite pressure is applied to both push buttons 14. As a result, the projections 15 disengage from the grooves 6 and 7 under the elastic action of the synthetic plastic material and increased pressure applied by the ear-shaped bent hooks 16 to the inclined surfaces 4 and 5. As a result, the pin 3 springs out of the housing cap.

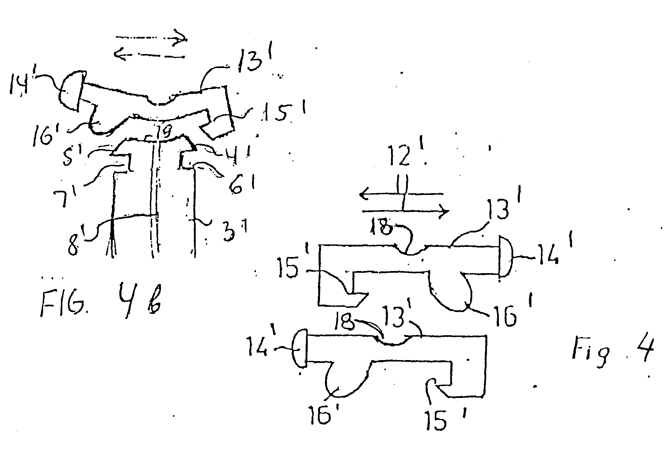

FIGS. 4A and 4B show the closure in accordance with a further embodiment of the present invention. The parts of this modification which are similar to the parts of the first embodiment are identified with the same reference numerals with added primes.

In the second embodiment of the invention there are also two sliders identified with reference numeral 13'. Each of the sliders has a push button 14', a projection 15', and a shaped formation 16' instead of the hook 16 of the first embodiment. Each of the sliders 13' is made elastic by a depression 18 provided on its upper surface, as can be seen from FIG. 4A. The pin 3' also has the inclined surfaces 4' and 5' and the grooves 6' and 7', as well as the longitudinal grooves 8'. In contrast to the first embodiment, the pin 3' has an upper surface 19 having a concave shape.

During assembly of the closure, the sliders 13' are arranged so that their lower surfaces are in contact with the upper concave surface 19 of the pin 3' and therefore the sliders 13' are bent and stressed. The projections 15' of the sliders of the housing cap engage in the grooves 6' and 7' of the pin 3' of the closure housing. On the other side, the formations 6' of the sliders 13' of the housing cap abut against the inclined surfaces 4' and 5' of the pin 3' of the closure housing. The arresting is performed in the bent condition of the sliders.

For releasing the housing cap from the pin 3' and therefore from the closure housing, an opposite pressure is applied to both push buttons 14'. As a result, the formations 16' slide along the inclined surfaces 4' and 5', the sliders 13' tend to assume their original straight shape, and the projections 15' disengage from the grooves 6' and 7' under the elastic action. As a result the pin 3' springs out of the housing cap.

While the invention has been illustrated and described as embodied in a closure for a turniquet for limbs, it is not intended to be limited to the details shown, since various modifications and structural changes may be made without departing in any way from the present invention.

Without further analysis, the foregoing will so fully reveal the gist of the present invention that others can, by applying current knowledge, readily adapt it for various applications without omitting features that, from the standpoint of prior art, fairly constitute essential characteristics of the generic or specific aspects of this invention.

## Claims

1. A closure for a band and the like, comprising a closure housing (1) adapted for passing a band (2) therethrough; a housing cap (9) adapted to be connected to an end of the band (2) and means for releasably connecting said closure housing (1) with said housing cap (9), said connecting means including a pin (3,3') projecting from the closure housing (1) and having a front end with two oppositely inclined surfaces (4,5,4',5') and two sliders (13,13') slidingly accommodated in said housing cap (9) so as to slide one over the other characterised in that the pin (3,3') has two transverse grooves (6,7,6',7') and in that each slider (13,13') has a first end provided with a push button (14,14'), a second end provided with a projection (15,15') and a shaped formation (16,16') situated between said projection (15,15') and said push button (14,14'), the part situated between said shaped formation (16,16') and said projection (15,15') or said shaped formation (16,16') itself being elastic such that in a locked condition said projections (15,15') of said sliders (13,13') are accommodated in said housing cap (9) and engage in said grooves (6,7,6',7') of said pin (3,3') of said closure housing (1) while said shaped formation (16,16') of said sliders (13,13') are accommodated in said housing cap (9), and abut against said inclined surfaces (4,5,4',5') of said pin (3,3') of said closure housing (1).

2. A closure as defined in claim 1, wherein said pin (3,3') of said closure housing (1) has a rectangular cross-section.

3. A closure as defined in claim 1, wherein said pin (3,3') has an end provided with said inclined surfaces (4,5;4',5') so that said inclined surfaces (4,5,4',5') together form a roof-shaped contour.

4. A closure as defined in claim 1, wherein said sliders (13,13') are composed of a synthetic plastic material.

5. A closure as defined in claim 1, wherein said shaped formations (16,16') of said sliders (13,13') of said housing cap (9) are elastically deformable hooks.

6. A closure as defined in claim 1, wherein said shaped formation (16,16') of said sliders (13,13') of said housing cap (9) are formed as solid elements.

7. A closure as defined in claim 1, wherein each of said sliders (13,13') of said connecting means has one end provided with said projection (15,15') and another end provided with a push button (14,14'), said projection (15,15') extends toward said push button (14,14') of each of said sliders (13,13').

8. A closure as defined in claim 7, wherein said shaped formation (16,16') of each of said sliders (13,13') is located between said projection (15,15') and said push button (14,14') of said slider.

9. A closure as defined in claim 1, wherein each of said sliders (13') has means imparting elasticity to said sliders (13'), said pin (3') aving an end surface (19) which is concave so that in an assembled condition said sliders (13') abut against said concave end surface (19) and are bent with a prestress.

10. A closure as defined in claim 9, wherein said imparting means includes at least one depression (18) provided in each of said sliders (13').

## Patentansprüche

1. Ein Verschluß für ein Band und dergleichen, bestehend aus einem Verschlußgehäuse (1) zum Durchziehen eines Bandes (2); einer Verschlußkappe (9), zur Verbindung mit einem Ende des Bandes (2); sowie Vorrichtungen für eine angemessene Verbindung des obenaufgeführten Verschlußgehäuses (1) mit besagter Gehäusekappe (9), wobei die besagten Verbindungsvorrichtungen einen Stift (3,3') beinhalten, der aus dem Verschlußgehäuse (1) herausragt, und desse Vorderende zwei entgegengesetzte Schrägflächen (4,5; 4', 5') und zwei Schieber (13, 13') aufweist, die gleitend in obenaufgeführte Gehäusekappe (9) eingeführt werden, so daß sie übereinander gleiten, und die dadurch gekennzeichnet sind, daß der Stift (3,3') zwei Querrillen (6,7; 6'7') aufweist, und daß jeder Schieber (13, 13') ein erstes Ende mit einem Druckknopf (14, 14') hat, und ein zweites Ende mit einem Vorsprung (15, 15') sowie einem Formteil (16, 16') zwischen obenaufgeführtem Vorsprung (15, 15') und besagtem Druckknopf (14, 14'), wobei das Teil zwischen dem besagten Formteil (16, 16') sowie besagtem Vorsprung (15, 15') bzw. besagtem Formteil (16, 16') selbst elastisch ist, so daß bei Verschluß die besagten Vorsprünge (15, 15') der besagten Schieber (13, 13') in besagter Gehäusekappe (9) liegen und in die besagten Rillen (6, 7; 6', 7') des besagten Stiftes (3, 3') des besagten Verschlußgehäuses (1) einrasten, während das besagte Formteil (16, 16') der besagten Schieber (13, 13')in besagter Gehäusekappe (9) liegt und an den Schrägflächen (4, 5; 4', 5') des besagten Stiftes (3, 3') des besagten Verschlußgehäuses (1) anliegt.

2. Ein Verschluß gemäß Anspruch 1, wobei der besagte Stift (3, 3') des besagten Verschlußgehäuses (1) einen rechteckigen Querschnitt hat.

3. Ein Verschluß gemäß Anspruch 1, wobei der besagte Stift (3,3') ein Ende mit den besagten Schrägflächen (4, 5; 4', 5') hat, so daß die besagten Schrägflächen (4, 5; 4', 5') zusammen einen dachförmigen Umriß bilden.

4. Ein Verschluß gemäß Anspruch 1, wobei die besagten Schieber (13, 13') aus Kunststoff bestehen.

5. Ein Verschluß gemäß Anspruch 1, wobei die besagten Formteile (16, 16') der besagten Schieber (13, 13') der besagten Gehäusekappe (9) elastische verformbare Haken darstellen.

6. Ein Verschluß gemäß Anspruch 1, wobei die besagten Formteile (16, 16') der besagten Schieber (13, 13') der besagten Gehäusekappe (9) als feste Teile ausgebildet sind.

7. Ein Verschluß gemäß Anspruch 1, wobei die besagten Schieber (13, 13') der besagten Verbindungsvorrichtungen jeweils ein Ende mit besagtem Vorsprung (15, 15') sowie ein weiteres Ende mit einem Druckknopf (14, 14') haben und besagter Vorsprung (15, 15') bis zum besagten Druckknopf (14, 14') der beiden besagten Gleitstücke (13, 13') reicht.

8. Ein Verschluß gemäß Anspruch 7, wobei die besagten Formteile (16, 16') der besagten Schieber (13, 13') zwischen besagtem Vorsprung (15, 15') sowie besagtem Druckknopf (14, 14#) des besagten Schiebers liegen.

9. Ein Verschluß gemäß Anspruch 1, wobei jeder der besagten Schieber (13, 13') Vorrichtungen hat, welche Elastizität auf die besagten Schieber (13') übertragen, und besagter Stift (3') eine konkave Endfläche (19) hat, so daß, wenn zusammengebaut, die besagten Schieber (13') an besagter konkaver Endfläche (19) anliegen und durch Vorspannung gebogen werden.

10. Ein Verschluß gemäß Anspruch 9, wobei die besagten übertragenden Vorrichtungen zumindest eine Vertiefung (18) an jedem der besagten Schieber (13') beinhalten.

## Revendications

1. Un fermoir pour une bande ou autre, comprenant un boîtier de fermoir (1) conçu pour que l'on puisse faire passer une bande (2) à travers lui; un chapeau de boîtier (9) conçu pour être fixé à une extrémité de la bande (2), et des moyens pour accoupler de façon libérable le boîtier de fermoir (1) et le chapeau de boîtier (9), ces moyens d'accouplement comprenant un doigt (3, 3') qui fait saillie à partir du boîtier de fermoir (1) et qui comporte une extrémité avant avec deux surfaces inclinées de façon opposée (4, 5; 4', 5') et deux éléments glissants (13, 13'), logés de façon glissante dans le chapeau de boîtier (9), de façon à glisser l'un sur l'autre, caractérisé en ce que le doigt (3, 3') comporte deux rainures transversales (6, 7; 6', 7') et en ce que chaque élément glissant (13, 13') comporte une première extrémité munie d'un bouton-poussoir (14, 14'), une seconde extrémité munie d'une saillie (15, 15'), et une structure profilée (16, 16') située entre la saillie précitée (15, 15') et le bouton-poussoir (14, 14'), la partie située entre la structure profilée (16, 16') et la saillie (15, 15'), ou la structure profilée (16, 16') elle-même, étant élastique, de façon que dans une condition verrouillée les saillies (15, 15') des éléments glissants (13, 13') soient logées dans le chapeau de boîtier (9) et pénètrent dans les rainures (6, 7; 6', 7') du doigt (3, 3') du boîtier de fermoir (1), tandis que les structures profilées (16, 16') des éléments glissants (13, 13') sont disposées dans le chapeau de boîtier (9) et portent contre les surfaces inclinées (4, 5; 4', 5') du doigt (3, 3') du boîtier de fermoir (1).

2. Un fermoir défini dans la revendication dans la revendication 1, dans lequel le doigt (3, 3') du boîtier de fermoir (1) a une section droite rectangulaire.

3. Un fermoir défini dans la revendication 1, dans lequel le doigt (3, 3') a une extrémité qui est munie des surfaces inclinées (4, 5; 4', 5'), de façon que ces surfaces inclinées (4, 5; 4', 5') forment ensemble un profil en forme de toit.

4. Un fermoir défini dans la revendication 1, dans lequel les éléments glissants (13, 13') sont constitués par une matière plastique synthétique.

5. Un fermoir défini dans la revendication 1, dans lequel les structures profilées (16, 16') des éléments glissants (13, 13') du chapeau de boîtier (9) sont des crochets déformables de façon élastique.

6. Un fermoir défini dans la revendication 1, dans lequel les structures profilées (16, 16') des éléments glissants (13, 13') du chapeau de boîtier (9) sont réalisées sous la forme d'éléments pleins.

7. Un fermoir défini dans la revendication 1, dans lequel une extrémité de chacun des éléments glissants (13, 13') des moyens d'accouplement est munie de la saillie précitée (15, 15'), et son autre extrémité est munie d'un bouton-poussoir (14, 14'), la saillie (15, 15') s'étendant vers le bouton-poussoir (14, 14') de chacun des éléments glissants (13, 13').

8. Un fermoir défini dans la revendication 7, dans lequel la structure profilée (16, 16') de chacun des éléments glissants (13, 13') se trouve entre la saillie (15, 15') et le bouton-poussoir (14, 14') de l'élément glissant considéré.

9. Un fermoir défini dans la revendication 1, dans lequel chacun des éléments glissants (13') comporte des moyens qui communiquent une élasticité aux éléments glissants (13') et le doigt (3') comporte une surface d'extrémité (19) qui est concave, de façon que dans une condition assemblée, les éléments glissants (13') portent contre la surface d'extrémité concave (19) et soient courbés avec une précontrainte.

10. Un fermoir défini dans la revendication 9, dans lequel les moyens communiquant une élasticité comprennent au moins un creux (18) formé dans chacun des éléments glissants (13').
